Europäisches Patentamt

European Patent Office

Office européen des brevets

(11) Publication number: **0 513 459 A1**

## EUROPEAN PATENT APPLICATION

(21) Application number: **91401272.9**

(22) Date of filing: **16.05.91**

(51) Int. Cl.5: **A61L 29/00**

(43) Date of publication of application:
**19.11.92 Bulletin 92/47**

(84) Designated Contracting States:
**BE DE FR GB IT NL SE**

(71) Applicant: **TERUMO KABUSHIKI KAISHA**
**No. 44-1, Hatagaya 2-chome, Shibuya-ku**
**Tokyo 151(JP)**

(72) Inventor: **Kousai, Tadashi, c/o Terumo K.K.**
**150 Maimaigi-cho, Fujinomiya-shi**
**Shizuoka(JP)**
Inventor: **Mihara, Nobuaki, c/o Terumo K.K.**
**150 Maimaigi-cho, Fujinomiya-shi**
**Shizuoka(JP)**

(74) Representative: **Gillard, Marie-Louise et al**
**Cabinet Beau de Loménie 55, Rue**
**d'Amsterdam**
**F-75008 Paris(FR)**

(54) Indwelling catheter.

(57) The present invention is the indwelling catheter which is inserted into a blood vessel, and is made of a material having a Shore D hardness of 50 through 70, and an impact resilience of 40% or more. The material of said catheter is a polyester elastomer having a hard segment comprising an aromatic crystalline polyester and an elastic segment comprising an aliphatic polyester or an aliphatic polyether.

F i g. 1

BACKGROUND OF THE INVENTION

Field of the Invention

The present invention related to an indwelling catheter suitable for use in liquid infusion, blood transfusion, or external circulation of blood.

Description of Related Art

An indwelling catheter comprises a catheter to be inserted into a blood vessel.

In a conventional indwelling catheter, fluororesin, polyurethane or the like is used as a material for said catheter. The inserting operability of such a conventional indwelling catheter has been improved by setting the hardness of a catheter material comparatively high to ensure the firmness of a catheter in its insertion into a blood vessel.

In such prior arts, however, there are problems (1) ~ (4) mentioned below.

(1) Since the anti-kink property (a property hard to be broken to the axial force) of a catheter material is not satisfied, a catheter may be broken when it is inserted into a blood vessel. Namely, its inserting operability is not satisfied.

(2) Since the impact resilience of a catheter material is not satisfied, the bend kink given to a catheter in the indwelling stage can not be immediately straightened, when it is removed from a blood vessel. And as a result, the catheter can not be smoothly removed from a region where it is has been indwelt. Namely, its removing operability is not satisfied.

(3) Since the impact resilience of a catheter material is not satisfied, a catheter can not smoothly follow the bend of a region where it will be indwelt, caused by the motion of an arm in its indwelling into a blood vessel. Namely, its indwelling stability is not satisfied.

Since the anti-kink property of a catheter material is not satisfied, a catheter may be broken by the strong bend of a region where it will be indwelt, caused by the motion of an arm in its indwelling into a blood vessel. Namely, its indwelling stability is not satisfied.

(4) Since the hardness of a catheter material is comparatively high, a large stimulation will be given on a blood vessel wall when a catheter is indwelt into a blood vessel, so that some damage of the blood vessel wall or Phlebitis may be caused. Namely, its biocompatibility is not satisfied.

(5) As to the indwelling catheter, there is recently seen a tendency to require an anti-thrombus property lasting for a given period of time or more, in addition to the above required characteristics. In the case of indwelling catheter to be indwelt in a patient continuously for a week or more, for instance, they have a tendency to seek a better biocompatibility, as compared with indwelling catheter made of conventional resin materials. Indwelling catheter have been, therefore, desired which exhibit a better anti-thrombus property, and which have further a "biocompatibly" indwelling stability, in addition to the inserting operability, removing operability and "mechanically" indwelling stability mentioned above.

SUMMARY OF THE INVENTION

The present invention is intended to provide an indwelling catheter excellent in the inserting operability, removing operability, indwelling stability and biocompatibility.

An indwelling catheter according to the invention described in claim 1 is inserted into a blood vessel, and is made of a material having a Shore D hardness of 50 throuh 70, and an impact resilience of 40 % or more.

In an indwelling catheter according to the invention described in claim 2, the material of said catheter is a polyester elastomer.

In an indwelling catheter according to the invention described in claim 3, said polyester elastomer is provided with a hard segment comprising an aromatic crystalline polyester and a elastic segment comprising an aliphatic polyester.

In an indwelling catheter according to the invention described in claim 4, said polyester elastomer is provided with a hard segment comprising an aromatic crystalline polyester and a elastic segment comprising an aliphatic polyether.

In addition, the "impact resilience" is defined by Japanese Industrial Standard JIS K7311.

According to the invention described in claim 1, the following effects (1) to (4) will be obtained.

(1) If the hardness of a catheter material is Shore D of less than 50, it is impossible to ensure the firmness required for a catheter. According to the present invention, namely, the hardness of a catheter material is Shore D of over 50, thereby ensuring the firmness required for a catheter. Namely, an excellent inserting operability can be ensured.

(2) If the impact resilience of a catheter material is less than 40 %, a catheter will be easily plastically deformed, with its recovery reduced, and it is further easily given a bend kink. According to the present invention, namely, the impact resilience of a catheter material is over 40 % and it is satisfied, and a catheter hardly be, therefore, given any bend kink, and can smoothly follow the bend of a region where it will be indwelt, caused by the motion of an arm in its indwelling into a blood vessel. Namely, an excellent indwelling satability can be ensured.

(3) Since the impact resilience of a catheter material is satisfied as mentioned in the above item (2), a catheter hardly be given any bend kink and the bend kink given to the catheter in the indwelling stage can be immediately straightened, when it is removed from a blood vessel, and as a result, the catheter can be smoothly removed therefrom. Namely, an excellent removing operability can be ensured.

(4) Since the hardness of a catheter material is Shore D of below 70 and it is sufficiently flexible, only a small stimulation will be given on a blood vessel wall, when a catheter is indwelt into a blood vessel, so that any damage of the blood vessel wall or phlebitis may not be caused. Namely, an excellent biocompatibility iy can be ensured.

According to the invention described in claim 2, 3 and 4, the following effects (5), (6) and (7) will be obtained.

(5) Since a catheter material is polyester elastomer, the anti-kink property of a catheter is satisfied and the catheter may not be, therefore, broken when it is inserted into a blood vessel. Namely, an excellent inserting operability can be ensured.

(6) Since the anti-kink property of a catheter material is satisfied as mentioned in the above item (5), a catheter may not be broken, even by the strong bend of a region where it will be indwelt, caused by the motion of an arm in its indwelling into a blood vessel. Namely, an excellent indwelling stability can be ensured.

(7) Since a catheter material is polyester elastomer, the catheter exhibit a better anti-thrombus property, and has further a "biocompatibly" indwelling stability.

BRIEF DESCRIPTION OF THE DRAWINGS

Fig. 1 is a schematic view showing an indwelling catheter assembly, to which the present invention is applied ; and
Fig. 2 is a schematic view showing the recovery test of an indwelling catheter.

DETAILED DESCRIPTION OF THE PREFERRED EMBODIMENTS

An indwelling catheter assembly 1 is assembled by incorporating a puncture needle 20 into an indwelling catheter 10.

The indwelling catheter 10 is made up by bonding a catheter 12 to a hub 11. The puncture needle 20 is made up by bonding a hollow stainless needle 22 to a hub 21. The hub 21 of the puncture needle 20 has a filter 23 put thereon.

The indwelling catheter 10 is introduced into a blood vessel in the state of being incorporated with the puncture needle 20. After the introduction of the indwelling catheter 10 into the blood vessel is confirmed by virtue of the coloration of the filter 23 due to blood, the puncture needle 20 is separated for removal therefrom, and an infusion tube will be connected to the hub 11. Thus, the indwelling catheter 10 is indwelt in such a state that the catheter 12 is further introduced into the blood vessel, and then removed from the blood vessel, after it is used for infusion for a given period of time.

The catheter 12 of the indwelling catheter 10 is, indeed, made of a material having a Shore D hardness of 50 through 70 and an impact resilience of 40% or more.

In the present invention, polyester elastomers are used as a polymer meterial for said catheter 12, because these polymers have a proper biocompatibility as well as the abovementioned hardness and impact resilience.

Polyester elastomers are of block copolymers in which the chemically structural hard segment comprises a polyester component. In general, an aromatic crystalline polyester compnent is used for the hard segment. Polyester elastomers are divided, according to the chemical structure of their elastic segment, into two types. Namely, one is a polyester-polyester type in which the elastic segment comprises an aliphatic polyester componet that is elasticer and has a lower glass transition temperature, and the other is a polyester-polyether type in which the elastic segment comprises an aliphatic polyether component.

Both of them can be used in the present invention, because as to both the types of the polyester elastomers, it is possible to prepare optional polymers, from one being elastic to one having a rigidity, by properly combining the proportions of a hard segment and a elastic segment. When the proportion of a elastic segment is reduced, for instance, the characteristics of a resulting polyester elastomer are in that the hardness becomes higher, the heat resistance, oil resistance and mechanical strength are improved, and the rubber elasticity is simulataneously decreased.

The polyester elastomers which can be preferably used in the present invention may be polyester-ether types, and the following can be further preferably used.

HYTREL (registered trademark) made by Toray-Du Pont Co., Ltd.

PERPREN (registered trademark) made by Toyobo Co., Ltd.

ARNITEKL (registered trademark) made by AKZO Co., Ltd.

The reason for each of the abovementioned limitations on the catheter 12 will be described in detail.

(A)-(1) If the hardness is Shore D of less than 50, it is impossible to ensure an excellent inserting operability, because the firmness of a catheter which is required for its insertion into a blood vessel can not be ensured.

(A)-(2) If the hardness is Shore D of over 70, it is impossible to ensure an excellent biocompatibility, because a large stimulation will be given on a blood vessel wall, when a catheter is indwelt into a blood vessel.

(B) If the impact resilience is less than 40 %, it is impossible to ensure an excellent indwelling stability and removing operability, because a catheter is plastically deformed, with its recovery reduced, and it is easily given same bend kink (See: Table 1).

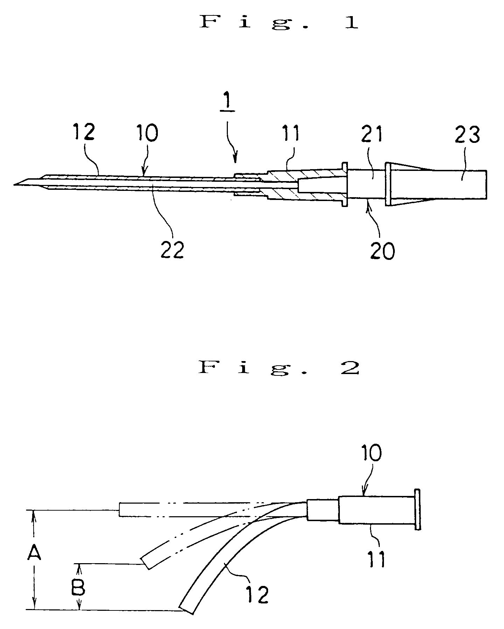

The table 1 shows the results of the recovery test illustrated in Fig. 2. In this recovery test, after a catheter, 2 inches in length, 1.1 mm in outer diameter and 0.78 mm in inner diameter was left, for five minutes, in an initial state in that its distal end was bent by A = 40.0 mm, a return B after an elapse of given time was measured and a recovery was calculated, as follows:

$$\text{Recovery} = B/A \times 100 \ (\%)$$

And, the bend kink was evaluated by the resulting recovery.

In each of the Inventive Examples and the Comparative Examples in Table 1, the following materials are used for the polyester elastomer:

| Examples | Types of resin materials (Name of the materials) |
|---|---|
| Inventive Example 1 | Aromatic polyester-aliphatic polyether type (HYTREL (registered trademark) 5577; made by Toray-Du Pont Co., Ltd.) |
| Inventive Example 2 | Aromatic polyester-aliphatic polyether type (HYTREL (registered trademark) 6377; made by Toray-Du Pont Co., Ltd.) |
| Comparative Example 1 | Fluororesin ETFE (Polyethylene/tetrafluoroethylene copolymer) (AFLON (registered trademark) COP C-88AX; made by Asahi Glass Co., Ltd.) |
| Comparative Example 2 | Thermoplastic polyurethane elastomer (Thermoplastic polyether type polyurethane*) (Vialon (registered trademark) resin** ; made by Becton, Dickinson and Company) |

*: diphenylmethane diisocyanate/1,4-butandiol/polytetramethylene-etherglycohol
**: a resin for DESEREP Insyte (registered trademark) indwelling catheter, made by Becton, Dickinson and Company.

(C) In a case polyester elastomer is used, the anti-kink property (a property hard to be broken to the axial force) can be improved as compared with the cases using fluororesin or polyurethane. Namely, an excellent inserting operability and indwelling stability can be ensured.

(D) Evaluation of the biocompatibility

The time until a blood was coagulated using a rabbit blood was measured by the Chandler loop method. As a result, the polyester elastomers used in Inventive Examples 1 and 2 exhibited a better biocompatibility than the fluororesin used in Comparative Example 1 shown in Table 2. Furthermore, these polyester elastomers exhibited better results, as compared with Pellethane Type 2363-D55, made by MD Kasei Co., Ltd. (Comparative Example 3) which is of the same kind of thermoplastic polyurethane as the thermoplastic polyurethane elastomer used in Comparative Example 2, and which is of polyurethane elastomer usable for a medical material.

The effects of the abovementioned embodiments will be described.

(1) If the hardness of a catheter material is Shore D of less than 50, it is impossible to ensure the firmness required for the catheter 12. According to the abovementioned examples, namely, an excellent inserting operability can be ensured, because the hardness of a catheter material is Shore D of over 50, thereby ensuring the firmness required for the catheter 12.

(2) If the impact resilience of a catheter material is less than 40 %, a catheter is easily plastically deformed, with its recovery reduced, and it is easily given same bend kink. According to the abovementioned examples, namely, an excellent indwelling stability can be ensured, because the impact resilience of a catheter material is over 40 % and it is satisfied, and a catheter hardly be, therefore, given any bend kink, and can smoothly follow the bend of a region where it will be indwelt, caused by the motion of an arm in its indwelling into a blood vessel.

(3) Since the impact resilience of a catheter material is satisfied as mentioned in the above item (2), an excellent removing operability can be ensured, because a catheter hardly be given any bend kink, and the bend kink given to the catheter in the indwelling stage can be immediately straightened when it is removed from a blood vessel, and as a result, the catheter can be smoothly removed from a region where it has been indwelt.

(4) Since the hardness of a catheter material is Shore D of below 70 and it is sufficiently flexible, an excellent biocompatibility can be ensured, because only a small stimulation will be given on a blood vessel wall when a catheter is indwelt into a blood vessel so that any damage of the blood vessel wall or phlebitis may not be caused.

(5) Since a catheter material is polyester elastomer, an excellent inserting operability can be ensured, because the anti-kink property is satisfied and a catheter 12 may not be, therefore, broken when it is inserted into a blood vessel.

(6) Since the anti-kink property of a catheter material is satisfied as mentioned in the above item (5), an excellent indwelling stability can be ensured, because a catheter 12 may not be broken even by the strong bend of a region where it will be indwelt, caused by the motion of an arm in its indwelling into a blood vessel.

(7) Since a catheter material is polyester elastomer, the catheter 10 exhibit a better anti-thrombus property, and has further a "biocompatibly" indwelling stability.

One of the preferred embodiments according to the present invention can be given which uses a polyester elastomer containing a contrast medium blended therein in the catheter portion of an indwelling catheter (at 12 of Fig. 1). If the untransmissibility of X-ray is given to a catheter portion, namely, the relative position of said catheter can be revealed through the fluoroscopy on a display device, or photographed on a X-ray film, by applying X-ray to a patient body or the like.

As to the contrast mediums to be blended in polyester elastomer, in addition, there can be typically given barium sulfate, bismuth trioxide, and bismuth subcarbonates and tungsten. The blending quantity of these contrast mediums to polyester elastomer is usually 5~ 50wt% in total, preferably 8 ~ 45wt%, and in particular most preferably 10 ~ 40wt%.

In a general embodiment, a contrast medium is blended so as to be uniformly dispersed in a catheter. According to the purpose, however, it may be unevenly dispersed positively. For a concrete example, there will be given such an embodiment that a contrast medium is blended so that one straight stripe or a stripe-pattern containing a plurality of stripes coaxially arranged is formed on a tube.

According to the pressent invention, as mentioned above, an indwelling catheter can be obtained which is excellent in the inserting operability, removing operability, indwelling stability, and biocompatibility.

Table 1

|  | Hardness | Impact Resilience (%) | Recovery (%) after 15sec. | Recovery (%) after 30sec. |
|---|---|---|---|---|
| Inventive<br>Example 1<br>Example 2 | Shore D 55<br>Shore D 63 | 50<br>45 | 92.5<br>90.0 | 95.0<br>93.8 |
| Comparative<br>Example 1<br>Example 2 | Shore D 75<br>Shore D 40 | below 10<br>40 | 75.0<br>75.0 | 90.2<br>84.5 |

Table 2

| | The time until a blood was coagulated. | | | | | |
|---|---|---|---|---|---|---|
| | No.1 | No.2 | No.3 | No.4 | No.5 | the mean |
| Inventive<br>Example 1<br>Example 2 | 11'16"<br>10'37" | 6'34"<br>7'47" | 5'05"<br>3'44" | 4'36"<br>4'58" | 4'37"<br>5'01" | 6'26"<br>6'25" |
| Comparative<br>Example 1<br>Example 3 | 9'35"<br>9'55" | 5'44"<br>4'43" | 3'18"<br>4'44" | 4'06"<br>3'36" | 3'34"<br>3'34" | 5'15"<br>5'14" |

( ' : minute, " : second )

**Claims**

1. An indwelling catheter for being inserted into a blood vessel, comprising : said catheter being made of a material having a hardness, Shore D of 50 through 70, and an impact resilience of 40% or more.

2. An indwelling catheter, as set forth in claim 1, in which the material of said catheter is a polyester elastomer.

3. An indwelling catheter, as set forth in claim 2, wherein said polyester elastomer is provided with a hard segment comprising an aromatic crystalline polyester and a elastic segment comprising an aliphatic polyester.

4. An indwelling catheter, as set forth in claim 2, wherein said polyester elastomer is provided with a hard segment comprising an aromatic crystalline polyester and a elastic segment comprising an aliphatic polyether.

Fig. 1

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 354 695 (BAXTER INTERNATIONAL)<br>* column 4, line 27 - line 53 *<br>--- | 1-2 | A61L29/00 |
| X | FR-A-2 651 681 (MEDICORP RESEARCH LABORATOIRES CORP.)<br>* page 1, line 19 - line 22 *<br>* page 1, line 35 - page 2, line 12 *<br>--- | 1-2 | |
| X | PLASTIQUES MODERNES ET ELASTOMERES<br>vol. 36, no. 7, September 1984, PARIS, FRANCE<br>pages 40 - 42;<br>MARIE-HELENE TEXIER: 'LES PEBA A L'HEURE DE LA MATURITE'<br>* page 41, last paragraph - page 42, paragraph 1; figures *<br>--- | 1-2 | |
| A | EP-A-0 295 055 (YISSUM RESEARCH DEVELOPMENT CO.)<br>* claims 1-8 *<br><br>----- | 1-4 | |
| | | | **TECHNICAL FIELDS SEARCHED (Int. Cl.5)**<br><br>A61M<br>A61L |

The present search report has been drawn up for all claims

| Place of search | Date of completion of the search | Examiner |
|---|---|---|
| THE HAGUE | 16 JANUARY 1992 | MIR Y GUILLEN V. |

EPO FORM 1503 03.82 (P0401)